# EUROPEAN PATENT APPLICATION

(11) **EP 3 697 104 A1**
(43) Date of publication of application: **19.08.2020**
(21) Application number: 20156012.5
(22) Date of filing: 07.02.2020
(51) Int. Cl.: H04R 1/12, H04R 25/00, A61L 2/00, F26B 9/00

(54) **HEARING AID MAINTENANCE SYSTEM**

(30) Priority: 15.02.2019 US 201962806175 P; 26.04.2019 US 201916395609
(71) Applicant: Schumaier, Daniel R., Johnson City, TN 37601 (US)
(72) Inventor: Schumaier, Daniel R., Johnson City, TN 37601 (US)
(74) Representative: Espiell Gomez, Ignacio

(57) **Abstract**

A hearing aid maintenance system including a housing for receiving a hearing aid to be charged and dried and a hearing aid charging port, a fan, and a heater for heating air to assist in drying the hearing aid. The system includes circuitry for controlling the hearing aid charging port to selectively charge the hearing aid, for controlling the heater to maintain a battery charging temperature within a predetermined range, and for controlling the fan to provide a flow of air to dry the hearing aid.

## Description

### SPECIFICATION

### RELATED APPLICATION

This application claims priority to provisional application No. 62/806,175, filed February 15, 2019.

### TECHNICAL FIELD

The disclosure is related to a maintenance system for hearing aid devices and in particular to a charging, drying, and disinfection station for chargeable hearing aid devices.

### BACKGROUND AND SUMMARY

A hearing aid patient relies on a hearing aid device, and thus its components, to reliably function. Hearing aid devices comprise numerous sensitive electronic components that require periodic maintenance. These components may include a receiver, microphone, volume control, potentiometers, contacts, rechargeable batteries, and circuitry.

Hearing aid devices are subject to a moist environment when worn by a user. Moisture alone may negatively impact device performance and longevity particularly with regard to the electronic components. Moisture also aggravates the buildup up of ear wax, dirt, and grime, which may also deteriorate performance and longevity.

Untreated moisture may, for example, cause corrosion on contacts, potentiometers, circuitry, batteries, and wires, condensation on screens or diaphragms in the microphone/receiver, and/or loss of sensitivity of or change in the frequency response of the microphone/receiver. Further, untreated moisture and buildup may lead to ear infections.

Reducing moisture content and/or facilitating the removal of buildup and bacteria assists in the reliable functionality, maintainability, cleanliness, and longevity of hearing aid devices and prevents unwanted ear infections.

Most conventional hearing aid cleaning devices are suitable for maintaining the cleanliness of hearing aids. However, conventional hearing aid maintenance systems are primarily directed to drying and/or disinfecting the hearing aids. Rechargeable hearing aids have an added need for battery recharging circuitry during the disinfection process. There is also a need for a hearing aid maintenance system that prevents overheating of the rechargeable battery during the recharging process. There is a need therefore for a hearing aid maintenance system that provides a number of useful maintenance procedures for hearing aids, including but not limited to, reduction of moisture, disinfection, and battery recharging. There is also a need for a complete electronic maintenance station that integrates the drying and recharging process for hearing aids while preventing overheating of the hearing aid batteries.

In view of the foregoing there is provided a hearing aid maintenance system including a housing for receiving a hearing aid to be charged and dried and a hearing aid charging port, a fan, and a heater for heating air to assist in drying the hearing aid. The system includes circuitry for controlling the hearing aid charging port to selectively recharge the hearing aid, for controlling the heater to maintain a battery charging temperature within a predetermined range, and for controlling the fan to provide a flow of air to dry the hearing aid.

In some embodiments, the housing includes a desiccant disposed in the housing and the housing is not vented to the atmosphere.

In some embodiments, the housing is devoid of a desiccant and the housing is vented to the atmosphere.

In some embodiments, the charging port includes mechanically biased charging pins for maintaining contact with battery recharging terminals on the hearing aid.

In some embodiments, the hearing aid charging port further includes a magnet for urging the hearing aid into a proper position in the charging port.

In some embodiments, the hearing aid charging port comprises a nearfield inductive charging circuit.

In some embodiments, the circuitry includes a processor for controlling the hearing aid charging port, the heater, and the fan.

In some embodiments, the system includes an ultraviolet (UV) disinfection unit that is controlled by the processor.

In some embodiments, the fan provides an air flow path through the housing so that air flows through the heater to heat and dry the hearing aid.

In some embodiments, the fan provides an air flow path through the housing so that the air flow through the desiccant and the heater to heat and dry the hearing aid.

In some embodiments, the fan is resistant to heavy magnetic fields due to the fan's orientation and position within the housing.

In some embodiments, the circuitry includes a charging circuit board that plugs into a main circuit board, wherein the hearing aid charging port is connected to the charging circuit board.

In some embodiments, the circuitry is powered by a USB power input.

In some embodiments, the housing includes a tray containing the charging port and the charging circuit board.

In some embodiments, the tray includes a plurality of holes for flow of air therethrough.

In some embodiments, the circuitry controls the heater to maintain a temperature within the housing in a predetermine temperature range known to be safe and effective for recharging lithium-ion batteries or silver-zinc batteries.

In some embodiments, the predetermined temperature range is between about 5ºC and about 45º C.

### BRIEF DESCRIPTION OF THE DRAWINGS

The invention will be further understood from the drawings herein of certain preferred embodiments, wherein the structures are not drawn to scale, and the following description thereof, wherein:
FIG. 1 is a perspective view, not to scale, of a hearing aid maintenance system according to the disclosure.
FIG. 2 is perspective view, not to scale, of the hearing aid maintenance system of FIG. 1 illustrating features of the system.
FIG. 3 is a perspective top view, not to scale, of a tray containing hearing aid charging ports for the hearing aid maintenance system of FIG. 1.
FIG. 4 is a perspective bottom view, not to scale, of the tray of FIG. 3.
FIG. 5 is a schematic illustration of air flow through the maintenance system of FIG. 1.
FIG. 6 is a schematic illustration of the recharging control circuit on a battery charging circuit board for the system of FIG. 1.
FIG. 7 is a schematic illustration of a main circuit board for the system of FIG. 1
FIG. 8 is a schematic illustration of a control system for the hearing aid maintenance system of FIG. 1.
FIG. 9 is a perspective view, not to scale, of a hearing aid maintenance system according to an alternative embodiment of the disclosure.
FIG. 10 is a schematic illustration of air flow through the maintenance system of FIG. 9.

### DESCRIPTION OF THE PREFERRED EMBODIMENTS

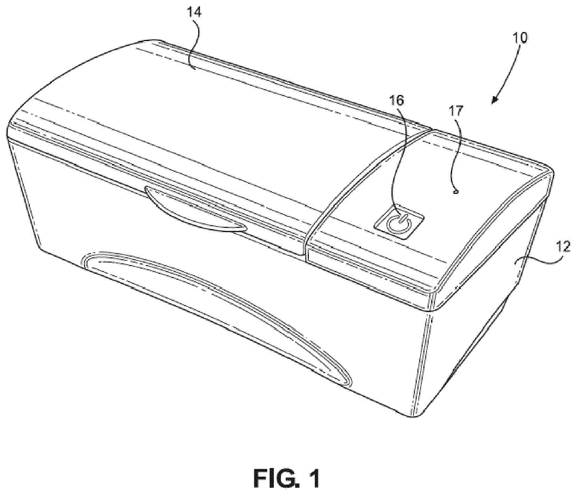

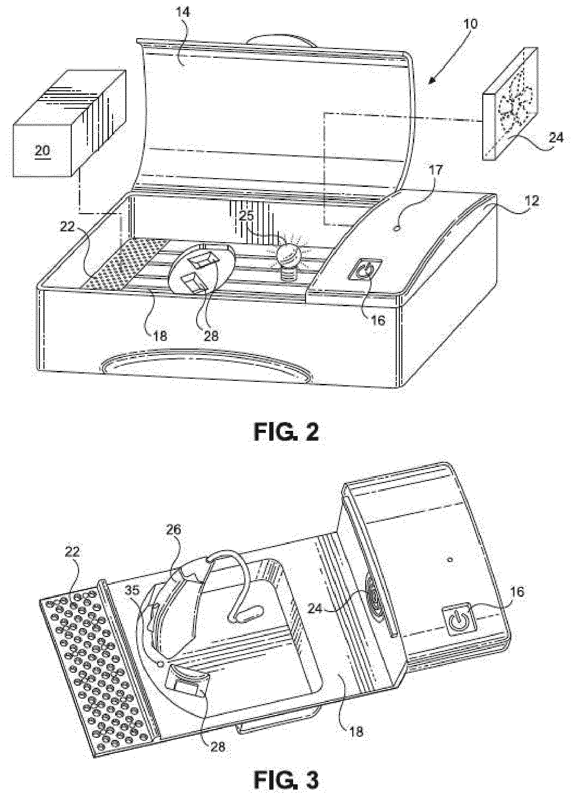

With reference to FIGS. 1 and 2, there is provided a hearing aid maintenance system 10 that provides a combination of hearing aid maintenance tasks in a convenient package. As shown, the overall configuration of the maintenance system 10 includes a lower portion in the shape of an open-top rectangular prism-shaped housing 12. A hinged cover 14 is attached to the housing 12 and a power button 16 is provided on the housing 12 to turn the system on and off. A particularly suitable power button 16 is a capacitive touch, power on/off switch. The capacitive touch switch eliminates the normal routine of common mechanical failures, such as wear out and contamination, which are leading causes of reduced life span in many products. An LED 17 is provided to indicate when power is provided to the system 10.

In one embodiment, as shown in FIG. 2, the housing 12 includes a tray 18 for holding a removable desiccant package 20 that contains desiccant for absorbing moisture. In a preferred embodiment, the desiccant package 20 is easily removed when saturated and may be dried at a higher temperature such as in an oven, and reused repeatedly in the system 10 or may be replaced with a fresh desiccant package 20. The desiccant package 20 may include any moisture absorbing material such as supported granular CaO, CaCl₂, ZnCl₂, CUSO₄, silica gel or the like. The package 20 is specifically designed to permit air flow through the package 20 in order to remove moisture from hearing aids 26. Accordingly, the tray 18 includes holes or apertures 22 for providing a source of air flow through the desiccant package 20. Air flow in the housing 12 may be provided by an internal fan 24 which is controlled, as described below, to provide drying air flow in the housing 12 when the cover 14 is closed. The desiccant package 20 may include an indicator, such as a humidity sensitive patch located on a visible portion thereof which undergoes a change in color, e.g., pink to blue as the desiccant material in the package 20 progressively loses its effectiveness. The desiccant package 20 preferably has a rectangular prism shape and is designed to provide adequate drying for about one month after which time the desiccant package 20 may be exchanged with a new desiccant package 20. When the desiccant package 20 is used in the system, the housing is preferably not vented to the atmosphere. In alternative embodiment, described in more detail below, the housing is vented to the atmosphere and does not contain a desiccant.

The fan 24 is internal to the housing 12 and preferably uses a magnetic bearing assembly instead of conventional ball bearings. The magnetic bearing assembly allows the hearing aid maintenance system 10 to operate with less operating motor noise, as well as extended life, and a higher level of reliability. A key design consideration for the fan 24 is to prevent implant products that use very strong magnetics from interfering with the operation of the fan 24. Accordingly, the hearing aid maintenance system 10, as described herein, is resistant to the heavy magnetic fields due to the location and orientation of the fan 24 as it relates to the drying area within the housing 12.

A germicidal lamp and/or ultraviolet (UV) lamp 25 may be provided in the housing 12 in the air flow stream provided by the fan 24 to sterilize surfaces of the hearing aids 26 (FIG. 3). When a germicidal lamp, such as a UV-C lamp 25, is used, direct irradiation of the hearing aids 26 directly kills bacteria or the like. Also, ozone may be produced by the UV-C lamp 25 to act as a deodorizer. A suitable germicidal lamp is a UV-C lamp 25 that is a high intensity 50 mm linear (253.7nm) germicidal lamp rated at 70uW/cm2. The UV-C lamp 25 preferably operates at the beginning of the power on cycle for 90 seconds, giving a LOG 4 theoretical kill rate of 99.99% of fungi and bacteria. The wavelength of 253.7 nanometers is proven to inhibit colony formation in microorganisms which may significantly reduce itching and infection of the ear canal. The hearing aid maintenance system 10 incorporates a lid open detection circuit, to turn off the UV-C lamp 25, in the event the lid is opened during the disinfection process.

As shown if FIG. 2, the tray 18 also includes one or more charging ports 28 for recharging the hearing aids 26. The charging ports 28 include contacts 32, such as pogo pins or other biased contacts, for making electrical connection to contacts on the hearing aids 26 for recharging the hearing aids 26. In other embodiments, magnetic contacts may be used. In one embodiment, magnets are provided in the bottom of each charging ports 28 to engage the hearing aid housings and urge the hearing aid housing into the appropriate position for making electrical connection with the contacts 32 for charging. FIG. 3 illustrates a hearing aid inserted into the charging port 28 for charging the hearing aid 26 while also drying and/or disinfecting the hearing aid. In a preferred embodiment, the tray 18 is large enough to hold hearing aids that are not rechargeable or that do not require charging during the drying and sterilizing operations. The tray 18 is easily replaced with alternate trays having different sized charging ports 28 for different size hearing aids and charging configurations. Accordingly, the tray 18 may be adapted to recharge hearing aids having voltages ranging from 1-volt DC to about 5-volts DC.

In some embodiments, one or more of the hearing aid charging ports 28 comprise a nearfield inductive charging circuit embedded in the tray 18, through which one or more hearing aids 26 are charged while disposed on top of or adjacent to the nearfield inductive charging circuit, thereby eliminate the need for electrical contacts.

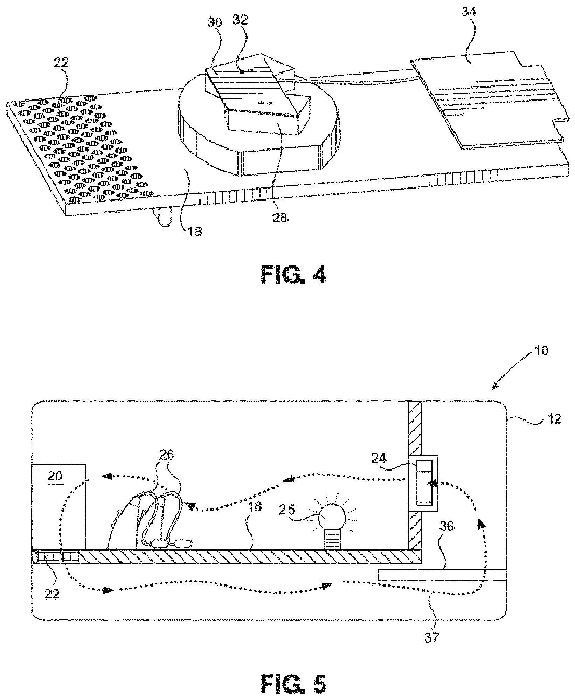

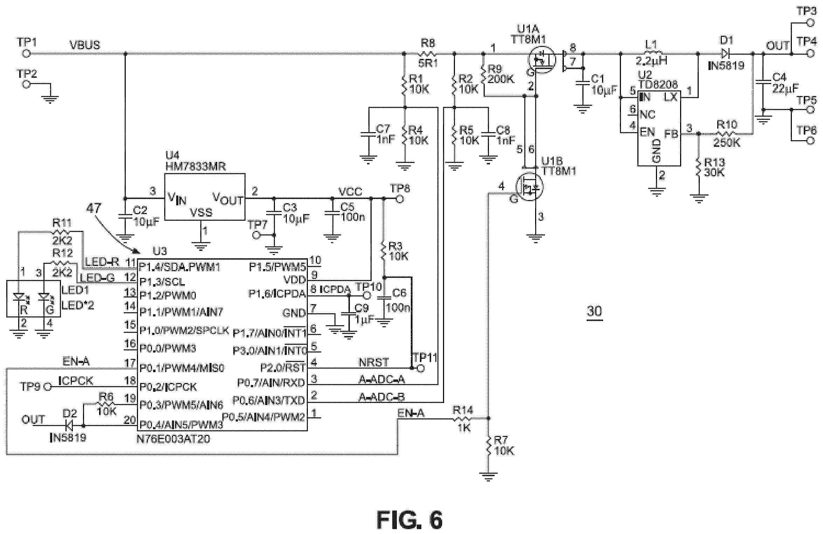

FIG. 4 provides an illustration of an underside of the tray 18. A circuit board 30 containing the contacts 32 is attached to the charging ports 28 for charging the hearing aids 26. The contacts 32 provide contact points for the hearing aids' lithium ion batteries or silver-zinc batteries. The circuit board 30 plugs into a main circuit board 34 that contains the capacitive touch switch electronics, a USB micro connector, a microcontroller, and a plug-in for the fan 24. The circuit board 30 is designed to maintain a charging voltage to the rechargeable hearing aids 26 in order to charge the hearing aids when power is applied to the system 10. An LED light 35 (FIG. 3) provides an indication of when charging is complete. Once the light 35 turns green, the hearing aids are charged and the charger will terminate the charging step. When the system 10 has power, a charging voltage is constantly applied to the contacts 32 so that the hearing aids 26 may be charged with and without the dryer system operating. A schematic drawing of the charging circuit board 30 is illustrated in FIG. 6. In a preferred embodiment, the circuit board 30 circuitry prevents overcharging of the hearing aid batteries. The charging circuit board 30 is integrated with the main circuit board 34 to provide temperature control of the rechargeable batteries during the charging and/or drying process.

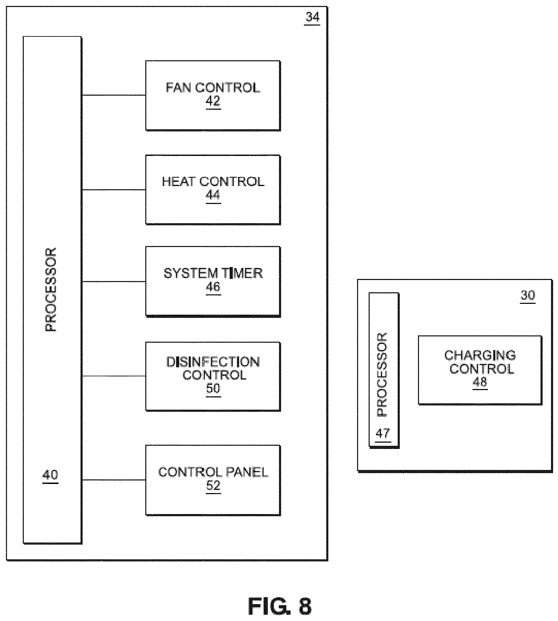

The system 10 also includes a heating element 36 (FIG. 5) that may be controlled by a main processor 40 (FIG. 8) for maintaining a constant temperature ideal for drying hearing aids 26. In a preferred embodiment, the heating element 36 comprises a miniature electrical grid or resistance heater wherein spacings between resistance wires allow air to flow through the heating element 36 to maintain an air flow circulation path in the system 10. A schematic drawing of an air flow path 37 through the maintenance system 10 is illustrated in FIG. 5.

In a preferred embodiment, the fan 24 is suitably mounted upstream of the desiccant package 20, and the heating element 36 is mounted between the desiccant package 20 and the fan 24 so that the heating element 36, UV-C lamp 25 and desiccant package 20 are all within an air flow circulation path of the fan 24. In some embodiments, the system 10 may also include replaceable chemical means such as alcohol-containing or antibiotic-containing slow release pads, or ejector means for atomizing or spraying germicidal or anti-fungal material into the air flow circulation path of the fan 24.

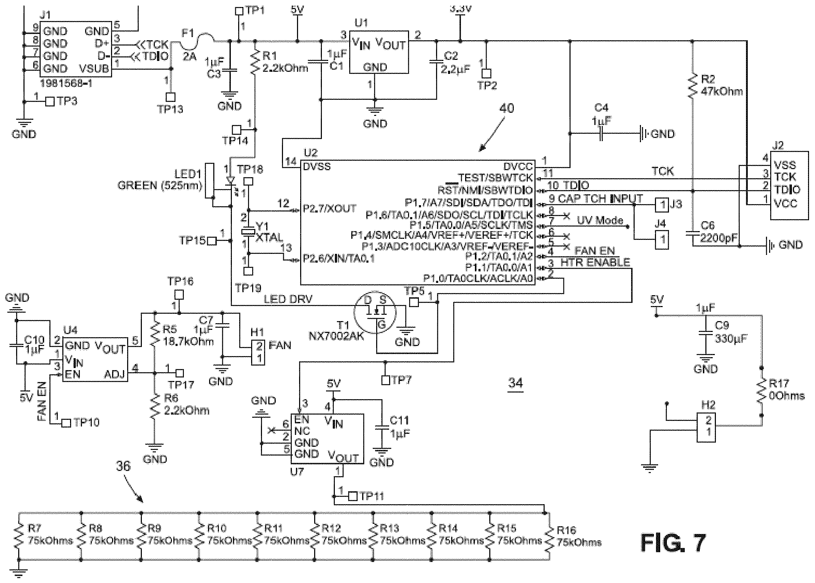

Referring to FIGS. 7 and 8, the system 10 preferably includes the main circuit board 34 containing the main processor 40 for operating the system 10. The main processor 40 includes a non-volatile memory 41 and control logic 42 for controlling the fan 24, heater control logic 44, system timer 46, disinfection control logic 50, and control panel logic 52. In a preferred embodiment, the circuit board 30 is electrically connected to the main circuit board 34 and contains a separate processor 47 that includes a non-volatile memory 49 and charging control logic 48 for charging the hearing aid batteries. The main processor 40 firmware has been configured to meet specific user requirements. When the system is activated by the capacitive touch switch 16, the main processor 40 determines what devices will be activated. In the case of the fan control logic 42, the main processor 40 determines if the fan is needed to circulate dry air and/or to cool components of the system to a desired operating temperature. For example, a thermistor may be used to determine if the hearing aid batteries are overheating. If the hearing aid batteries reach a predetermined temperature, the fan control logic 42 will activate the fan to cool the hearing aid batteries. The main processor 40 also activates the heater control logic 44 to quickly bring the air temperature to an appropriate temperature for drying the hearing aids 26, which may be set by an internal digital thermostat. If for any reason the temperature drops below about 30º C or any other such desired low threshold value, the heating element 36 may be reactivated. A system timer 46 may be provided to control the overall length of time the heating and drying operation is active. A typical drying cycle is 8 hours.

In a preferred embodiment, when the hearing aids 26 to be charged are positioned on the contacts 32 in the charging ports 28, the charging control logic 48 is automatically activated by the processor 47 to charge the hearing aid batteries. The charging control logic 48 maintains a suitable charge current for the batteries and also monitors the battery temperatures to determine if the batteries are overheating. If the batteries are overheating, the charging operation is stopped until the batteries cool down to a suitable charging temperature or the fan 24 may be activated as described above to cool the hearing aid batteries. In some embodiments, the main processor 40 maintains the temperature within the housing 12 within a temperature range known to be safe and effective for charging lithium-ion batteries or silver-zinc batteries, such as a range of between about 5º C to about 45º C.

In a preferred embodiment, the main processor 40 also controls the operation of the disinfection lamp 25 (which may be a UV-D lamp) to sterilize the hearing aids during the drying operation. The disinfection lamp 25 is preferably controlled by the disinfection control logic 50.

Power may be provided to the system 10 by a conventional electrical power supply circuit or by a rechargeable battery system contained within the housing 12. In one embodiment, the power supply is a 5-volt DC power supply, such as provided by a USB micro power connector via a power supply or a computer USB port. Accordingly, the system 10 can operate from the USB port of a computer or from a separate USB power supply.

When the system 10 is activated the heater and fan start to gently circulate warm air for approximately 8 hours. The combination of precisely controlled temperature and moving air breaks the surface tension bond that water molecules have on the hearing aids 26, and the air circulating through the system 10 then pulls that moisture away, allowing the desiccant in the desiccant package 20 to capture the moisture molecules and lock both the moisture and odor away inside the package 20. After a month of use, the desiccant package 20 may be discarded and replaced with a new one to keep the moisture and odors- locked away - instead of inside of the hearing aids 26. The system 10 preferably has a built-in timer and will automatically turn off after eight hours. The hearing aids 26 will begin charging when the hearing aids 26 are placed on the magnetic contacts 32 and will charge the length of time needed to completely charge the batteries, depending on how much battery power is left. This function can be used with or without the drying process.

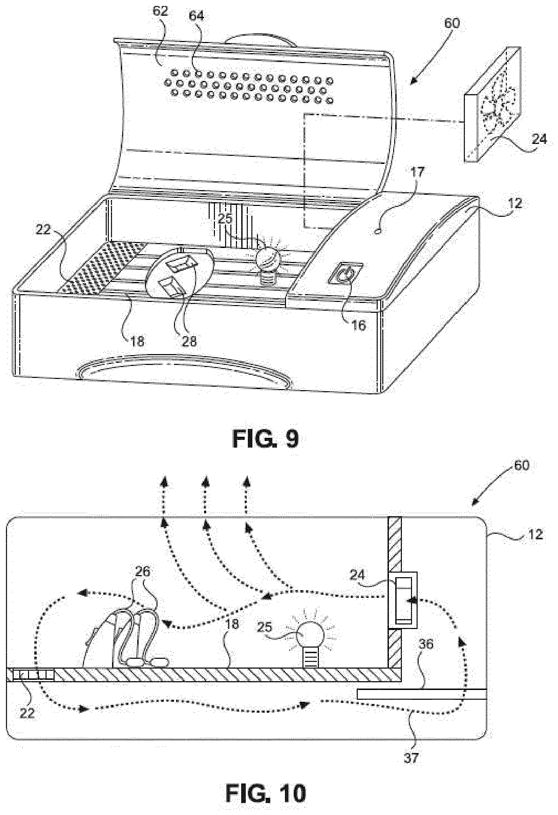

In an alternative embodiment, illustrated in FIGS. 9, the housing 12 is devoid of a desiccant package 20. Accordingly, a hearing aid maintenance system 60 has a cover 62 that contains vent holes 64 in order to vent the housing 12 to the atmosphere. In this embodiment, drying of the hearing aid 26 is achieved by circulating heated air using the fan 24 and heating element 36. A representative drying air flow path 66 is illustrated in FIG. 10. Moisture is removed from the hearing aids 26 through the cover 62 and vent hoes 64 by the circulation of heated air, in the absence of the desiccant.

Many of the structural components, including the housing 12, cover 14 and tray 18, may be made of durable plastic material.

The invention has been described in detail with particular reference to preferred embodiments thereof, but it will be understood that variations and modifications will be affected with the spirit and scope of the invention.

## Claims

1. A hearing aid maintenance system comprising:
a housing for receiving a hearing aid to be recharged and dried;
a hearing aid charging port disposed within the housing:
a fan disposed within the housing;
a heater disposed within the housing for heating air to assist in drying the hearing aid; and
circuitry for controlling the hearing aid charging port to selectively charge the hearing aid, for controlling the heater to maintain a battery charging temperature within a predetermined range, and for controlling the fan to provide a flow of air to dry the hearing aid.

2. The hearing aid maintenance system of claim 1, wherein the charging port further comprises mechanically biased pins for maintaining contact with battery charging terminals on the hearing aid.

3. The hearing aid maintenance system of claim 1, wherein the hearing aid charging port further comprises a magnet for urging the hearing aid into a proper position in the charging port.

4. The hearing aid maintenance system of claim 1, wherein the hearing aid charging port further comprises a nearfield inductive charging circuit.

5. The hearing aid maintenance system of claim 1, wherein the circuitry comprises a processor for controlling the hearing aid charging port, the heater, and the fan.

6. The hearing aid maintenance system of claim 5, further comprising an ultraviolet (UV) disinfection unit controlled by the processor.

7. The hearing aid maintenance system of claim 1, wherein the housing is not vented to the atmosphere and the housing further comprises a desiccant for drying the hearing aid.

8. The hearing aid maintenance system of claim 7, wherein the fan provides an air flow path through the housing so that air flows through the desiccant and the heater to heat and dry the hearing aid.

9. The hearing aid maintenance system of claim 8, wherein the housing further comprises a tray and the tray includes a desiccant holder for holding the desiccant, the desiccant holder having a plurality of holes adjacent to the desiccant for flow of air therethrough.

10. The hearing aid maintenance system of claim 1, wherein the housing is vented to the atmosphere and the housing is devoid of a desiccant.

11. The hearing aid maintenance system of claim 10, wherein the fan provides an air flow path through the housing so that air flows through heater to heat and dry the hearing aid and moist air is vented to the atmosphere through a cover on the housing.

12. The hearing aid maintenance system of claim 1, wherein the circuitry further comprises a charging circuit board that plugs into a main circuit board, wherein the hearing aid charging port is connected to the charging circuit board.

13. The hearing aid maintenance system of claim 12, wherein the housing further comprises a tray containing the charging port, and the charging circuit board.

14. The hearing aid maintenance system of claim 1, wherein the circuitry controls the heater to maintain a temperature within the housing in a predetermined temperature range known to be safe and effective for charging lithium-ion batteries or silver-zinc batteries.

15. The hearing aid maintenance system of claim 14, wherein the predetermined temperature range is between about 5º C to about 45º C.
